# EUROPEAN PATENT SPECIFICATION

(11) **EP 3 519 061 B1**
(45) Date of publication and mention of the grant of the patent: **29.03.2023**
(21) Application number: 17780049.7
(22) Date of filing: 25.09.2017
(51) Int. Cl.: A61Q 19/06, A61K 8/49, A61K 8/97

(54) **TOPICAL SLIMMING COSMETIC TREATMENT**
TOPISCHE KOSMETISCHE ABNEHMBEHANDLUNG
TRAITEMENT COSMETIQUE TOPIQUE AMINCISSANT

(30) Priority: 27.09.2016 FR 1659082
(43) Date of publication of application: 07.08.2019
(73) Proprietor: Sederma, 78610 Le Perray en Yvelines (FR)
(72) Inventor: MONDON, Philippe, 92120 Montrouge (FR)
(86) International application number: PCT/EP2017/074265
(87) International publication number: WO 2018/060148

(56) References cited:
- CN-A- 103 183 654
- JP-A- H0 469 314
- JP-A- H08 188 528
- JP-A- 2006 036 682
- US-A1- 2006 165 619
- US-A1- 2009 098 217
- US-A1- 2015 150 770
- DATABASE GNPD [Online] MINTEL; July 2001 (2001-07), "Firming Whipped Body Cream", XP002766291, Database accession no. 102929

## Description

### TECHNICAL FIELD

The present invention relates to a topical slimming non therapeutical cosmetic treatment for the face and/or the body.

Cosmetics, hygiene and personal care, and dermo-pharmacy industries are concerned.

### BACKGROUND ART

The use of a slimming topical care is an essential ally in the aesthetic seeking of a beautiful silhouette and/or a harmonious contour of face, and there is thus constantly a need for new active agents, in particular molecules and plant extracts, adapted for topical cosmetic use capable of reducing and/or preventing the appearance of localized unsightly fat clusters, such as beads, dimples, cellulitis or the so-called "orange peel" skin aspect.

Adipose tissue, predominant in the energy balance in mammals, consists of an extracellular matrix, a dense capillary network and specific cells, the adipocytes. Its development is characterized by modifications of all the components of the hypodermic tissue with repercussions in the more superficial layers. A dermis thickness increase is thus observed, probably due to fluid congestion and infiltration of adipose lobules deforming the dermal/hypodermal junction. It is this adiposity which manifests itself by an alteration of the silhouette and the appearance of beads and dimples more or less unsightly and sometimes an appearance of "orange peel".

The weight development of adipose tissue, a direct mirror of the reserves of lipid energy, in which many mechanisms are involved, is in particular a function of the increase in size and number of adipocytes.

The mature adipocyte is designed to constitute a body energy reserve, closely controlled by multiple systems, in particular hormonal systems. This differentiated cell, which has lost its capacity for division, can either increase its storage capacity by increasing the amount of fat stored in the form of triglycerides (the lipogenesis), or inversely mobilize this stored fat *via* a mechanism leading to the release of fatty acids and glycerol and/or mono- and/or di-esters glycerol (the lipolysis). Under normal circumstances, there is a balance between lipogenesis and lipolysis in order to ensure the stability of the fat mass. If, for a variety of reasons, there is an imbalance between these two mechanisms, the triglycerides accumulate in the adipocytes.

Adipocyte precursor cells (pre-adipocytes) present in the adipose tissue can, under the effect of different stimuli, multiply and differentiate into adipocytes throughout life. It is referred to as differentiation or adipocyte conversion.

The pre-adipocyte, which is in the active growth phase, must escape from this cell cycle, and this growth stop is necessary in order for it to engage in the adipocyte conversion process. Between growth stop and differentiation beginning into adipocyte, nevertheless a single additional division, so called clonal expansion, occurs, thus increasing the pool of pre-adipocytes competent for differentiation.

During the terminal phase of differentiation, lipogenesis is strongly activated and all enzymes involved in triglyceride formation increase: acetyl-CoA, glycerol-3-phosphate dehydrogenase (G3DPH), glyceraldehyde-3-phosphate dehydrogenase (G-3-DPH) and fatty acid synthase (FAS). Thereafter come into play: the expression of insulin-specific receptors, adrenergic receptors and specialized proteins such as perilipine and vimentin (formation of lipid droplets), and the secretion of agents promoting the development of the fat mass (monobutyrin, angiogenic factors, angiotensinogen II).

The aim of the present invention is to provide a slimming non therapeutical cosmetic treatment capable, in particular, of regulating the proportion of fat in adipocytes and/or of regulating adipocyte differentiation in order *in fine* to prevent and/or regulate fat installation, *i.e.* having a lipolytic action and/or an action on lipogenesis.

### SUMMARY OF THE INVENTION

To this end, the present invention provides the use of an extract of *Apium graveolens* seeds obtained by CO₂ supercritical extraction comprising at least 50% by weight of a mixture of alkyl phthalides consisting of sedanenolide, sedanolide and 3-n-butylphthalide, for a topical cosmetic slimming treatment.

It has been unexpectedly discovered that alkyl phthalides derived from *Apium graveolens* seeds, act on lipolysis but also have anti-adipogenic properties by an antagonistic effect on adipocyte differentiation. *In vitro* tests demonstrating these properties are given below in the description.

The *Apium graveolens* plant, commonly called celery, is part of the family of Apiaceae or Umbelliferae, which includes many other edible plants such as carrot and coriander. This plant is not toxic; all parts of it can be consumed as raw or cooked vegetables. The seeds are also used as spices.

Italy is the country of origin, this plant being widespread in the world, especially in Europe, Egypt, Algeria, Ethiopia, Asia and India.

Celery seeds contain about 3% of volatile oil and 15% of non-volatile vegetable oil.

An essential oil extracted from celery seeds comprises terpenes (D-limonene > 60% and selinene 10-20%) and phthalides (1-4%). Among the predominant phthalides, there is the 3-n-butyl-phthalide, the sedanolide and sedanenolide (also called senkyunenolide A).

The nonvolatile part includes petroselinic, oleic, linoleic, myristic, palmitic, palmitoleic, stearic and myristoleic fatty acids.

The *Apium graveolens* therefore comprises potentially very many active compounds. As a medicinal plant, it is recommended since thousands of years for many properties: aphrodisiac, anthelmintic, antispasmodic, anti-inflammatory, carminative, diuretic, emmenagogue, laxative, sedative, stimulant and tonic. The plant can be used against asthma, bronchitis and rheumatism. The wild celery seeds are used in traditional medicine in India and other countries that have adopted the Ayurvedic traditions to heal, as a tranquilizer, antispasmodic, nerve tonic, diuretic and antirheumatic. The phthalides included in the *Apium graveolens* are described as active against cancer, high blood pressure and cholesterol. The world of living organisms and plants in particular represents a very important source of innovative active molecules for cosmetics and pharmaceuticals. To extract and concentrate them, there are different methods, and the composition of the extract obtained will obviously depend on the method and the operating conditions.

The extraction technique with supercritical CO₂ is already widely used in the food, pharmaceutical and perfumery industries. Gaseous CO₂, present in low concentrations in the air, is compressed so that it becomes liquid. It is then an excellent solvent for fat-soluble molecules. Inert, it reacts little with the extracted molecules and leaves no residue since it is evaporated at the end of process and captured to be reused.

An essential oil of celery, obtained by hydro-distillation, comprising in majority D-limonene has been proposed in cosmetics to treat pigmentation spots.

"Cosmetic treatment" means a treatment that addresses normal, healthy skin, said treatment being intended to improve its appearance and condition. Such treatment has no therapeutic aim. "Alkyl-phthalide" can encompass phthalides in which the 6-carbon ring is aromatic and hydrophthalides in which the 6-carbon ring has only 1 or 2 unsaturated bonds.

An alkyl-phthalide has therefore the following general formula I:

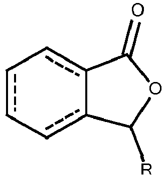

R being an alkyl chain of 1 to 8 carbon atoms, preferably 1 to 4, and preferably 4 (butyl phthalides), linear or branched, that can be substituted by an OH or amine function (secondary or tertiary).

Pure alkyl-phthalides can be obtained by chemical synthesis or by extraction and purification from a plant comprising them.

According to the invention, the used alkyl-phthalides comprise sedanenolide, sedanolide and 3-n-butylphthalide. These three alkyl-phthalides, shown thereafter, are those comprised in the volatile oil portion of the seeds of the *Apium graveolens* plant.

| | |
|---|---|
| 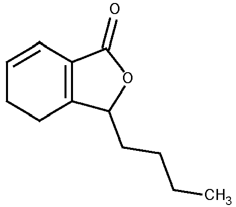 | Sedanenolide = |
| | Senkyunolide A = |
| | 3-butyl-6,7- dihydrophthalide |
| 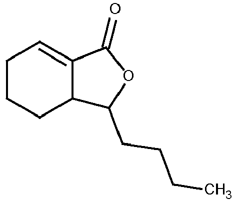 | Sedanolide |
| 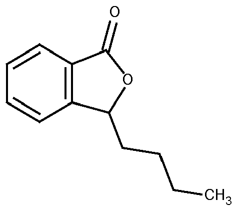 | 3-n-butylphthalide |

Thus, an extract *of Apium graveolens* seeds comprising sedanenolide, sedanolide and 3-n-butylphthalide is used according to the invention. Other plants may be used to extract one alkyl-phthalide according to the invention or produce an extract containing it as the major active component, particularly preferably one of the 3 alkyl-phthalides present in the seeds of *Apium graveolens.*

And according to the features of the invention an extract prepared by CO₂ supercritical extraction is used which provides an extract comprising at least 50% by weight of the alkyl-phthalides, preferably at least 65%, the remaining compounds mainly consisting of terpenes and traces of fatty acids. According to the invention the weight contents of these 3 phthalides based on total phthalides vary between:
Sedanolide: 0-45%
Sedanenolide: 45-90%
3-n-butylphthalide: 0-30%

Advantageously, an extract comprising preferably among the 3 alkyl-phthalides the sedanenolide as the major compound, preferably comprising at least 50%, and preferably at least 60%, and more preferably at least 70% of sedanenolide based on total phthalides, is used.

The extract is prepared by CO₂ supercritical extraction in a pressure range of 75 to 300 bars (75.10⁵ to 300.10⁵ Pa) and in a temperature range of 30°C to 80°C. Preferably the extraction will be done under 90 bars (90.10⁵ Pa) pressure and at 40°C.

Other extraction methods are disclosed to obtain an extract according to the invention comprising a majority of at least one alkyl-phthalide, including extraction with a nonpolar solvent such as hexane.

### DETAILED DESCRIPTION

### Preparation of compositions for implementing the present invention

Various types of compositions can be used for a slimming treatment according to the invention, such as, for example, a massage cream or oil, a face mask, a gel, a peelable adhesive product.

Each composition for a treatment according to the invention will comprise the alkyl phthalide plant extract in a physiologically acceptable medium which will differ according to the type of composition. "Physiologically acceptable medium" means according to the present invention, without limitation, an aqueous or hydro-alcoholic solution, a water-in-oil emulsion, an oil-in-water emulsion, a microemulsion, an aqueous gel, an anhydrous gel, a serum, a dispersion of vesicles, or a powder.

"Physiologically acceptable" means that the compositions are suitable for topical use, in contact with skin and scalp of mammals, particularly human, without risk of toxicity, incompatibility, instability, allergic response, and others.

This "physiologically acceptable medium" forms what is commonly called the excipient of the composition.

The alkyl-phthalide plant extract of the invention may be combined with other active ingredients at effective concentrations that can act synergistically or additionally for reinforcing and achieving the desired effects described for the invention, such as the following agents: moisturizing, antiaging, vitamins, radiation filters, in particular UVA and/or UVB, stimulating keratinocytes, antioxidants, antigycants, smoothing, toning.

The treatment of the invention may be applied to all body parts, and more specifically according to the preconized indication to the body and face, in whatever form or carrier known to those skilled in the art, in particular in the form of solution, dispersion, emulsion, paste, or powder, individually or as a premix or in vehicles individually or as a premix in vectors such as macro-, micro- or nano-capsules, macro-, micro- or , nano-spheres, liposomes, oleosomes or chylomicrons, macro-, micro-, or nanoparticles or macro-, micro- or nano-sponges, micro- or nano-emulsions or adsorbed on organic polymer powders, talcs, bentonites, spores or exines, and other inorganic or organic supports.

In general, the alkyl phthalide plant extract according to the invention may be used in any form, in a form bound to or incorporated in or absorbed in or adsorbed on macro-, micro-, and nanoparticles, or macro-, micro-, and nano-capsules, for the treatment of textiles, natural or synthetic fibres, wools, and any materials that may be used for clothing or underwear for day or night intended to come into contact with the skin, handkerchiefs or cloths like for example underpants or pantyhose, to exert their cosmetic effect via this skin/textile contact and to allow continuous topical delivery.

The CTFA (« International Cosmetic Ingredient Dictionary & Handbook » (16th Ed. 2016) published by « the Personal Care Products council », ex- « the Cosmetic, Toiletry, and Fragrance Association, Inc. », Washington, D.C.), describes a non-limited wide variety of cosmetic and pharmaceutical ingredients commonly used in the skin care industry, which are suitable for use as additional ingredients in the compositions according to the present invention.

Further additional skin care actives that are particularly useful can be found in the commercial literature of Sederma and on the website www.sederma.com.

The following commercial actives can also be mentioned, as examples: betaine, glycerol, Actimoist Bio 2^{™} (Active organics), AquaCacteen^{™} (Mibelle AG Cosmetics), Aquaphyline^{™} (Silab), AquaregulK^{™} (Solabia), Carciline^{™} (Greentech), Codiavelane^{™} (Biotech Marine), Dermaflux^{™} (Arch Chemicals, Inc), Hydra'Flow^{™} (Sochibo), Hydromoist L^{™} (Symrise), RenovHyal^{™} (Soliance), Seamoss^{™} (Biotech Marine), Argireline^{™} (commercial name for the acetyl hexapeptide-3 of Lipotec), spilanthol or an extract *of Acmella oleracea* known under the commercial name Gatuline Expression^{™}, an extract of *Boswellia serrata* known under the commercial name Boswellin^{™}, Deepaline PVB^{™} (Seppic), Syn-AKE^{™} (Pentapharm), Ameliox^{™}, Bioxilift^{™} (Silab), PhytoCellTec^{™}Argan (Mibelle), Papilactyl D^{™} (Silab), Preventhelia^{™} (Lipotec), and from Sederma: Subliskin^{™}, Venuceane^{™}, Moist 24^{™}, Vegesome Moist 24^{™}, Essenskin^{™}, Juvinity^{™}, Revidrat^{™}, Resistem^{™}, Chronodyn^{™}, Kombuchka^{™}, Chromocare^{™}, Calmosensine^{™}, Glycokin factor S^{™}, Biobustyl^{™}, Idealift^{™}, Ceramide 2^{™}, Ceramide A2^{™} et Ceramide HO3^{™}, Legance^{™}, Intenslim^{™}, Prodizia^{™}, Beautifeye^{™}, Pacifeel^{™}, NG-shea butter unsaponifiables (natural grade), Zingerslim^{™}, Meiritage^{™}, Senestem^{™}, Sebuless^{™}, Majestem^{™}, Rubistem^{™}, Citystem^{™}, or mixture thereof.

Among other plant extracts which can be combined with the alkyl phthalide plant extract of the invention, there may more particularly be mentioned extracts of Ivy, in particular English Ivy *(Hedera helix),* of *Bupleurum chinensis,* of *Bupleurum falcatum,* of arnica (*Arnica montana L),* of rosemary *(Rosmarinus officinalis N),* of marigold (*Calendula officinalis),* of sage (*Salvia officinalis L),* of ginseng (*Panax ginseng),* of ginko biloba, of St.-John's-Wort (*Hyperycum perforatum*), of butcher's-broom (*Ruscus aculeatus L),* of European meadowsweet (*Filipendula ulmaria L),* of big- flowered Jarva tea (*Orthosiphon stamincus benth*), of artichoke (*Cynara scolymus*), of algae (*Fucus vesiculosus*), of birch (*Betula alba),* of green tea, of cola nuts *(Cola nipida),* of horse-chestnut, of bamboo, of *Centella asiatica,* of heather, of fucus, of willow, of mouse-ear, of escine, of cangzhu, of *chrysanthellum indicum,* of the plants of the *Armeniacea* genus, *Atractylodis platicodon, Sinnomenum, Pharbitidis, Flemingia,* of *Coleus* such as *C. Forskohlii, C. blumei, C. esquirolii, C. scutellaroides, C. xanthantus* and *C. Barbatus,* such as the extract of root of *Coleus barbatus,* extracts of *Ballote,* of *Guioa,* of *Davallia,* of *Terminalia,* of *Barringtonia,* of *Trema,* of *antirobia, cecropia, argania, dioscoreae* such as *Dioscorea opposita* or Mexican, extracts of *Ammi visnaga,* of *Siegesbeckia,* in particular *Siegesbeckia orientalis,* vegetable extracts of the family of *Ericaceae,* in particular bilberry extracts (*Vaccinium angustifollium)* or *Arctostaphylos uva ursi, aloe vera,* plant containing sterols (e.g., phytosterol), Manjistha (extracted from plants of the genus *Rubia,* particularly *Rubia cordifolia*), and Guggal (extracted from plants of the genus *Commiphora,* particularly *Commiphora mukul),* kola extract, chamomile, red clover extract, Piper methysticum extract (Kava Kava^{™} from Sederma), *Bacopa monieri* extract (Bacocalmine^{™} from Sederma) and sea whip extract, extracts of *Glycyrrhiza glabra,* of mulberry, of *melaleuca* (tea tree), of *Larrea divaricata,* of *Rabdosia rubescens,* of *Euglena gracilis,* of *Fibraurea recisa Hirudinea,* of *Chaparral Sorghum,* of sun flower extract, of *Enantia chlorantha,* of Mitracarpe of *Spermacocea* genus, of *Buchu barosma, of Lawsonia inermis L.,* of *Adiantium capillus-veneris L.,* of *Chelidonium majus,* of *Luffa cylindrica,* of Japanese Mandarin (*Citrus reticulata Blanco* var. *unshiu*), of *Camelia sinensis,* of *Imperata cylindrica,* of *Glaucium Flavum,* of *Cupressus sempervirens,* of *Polygonatum multiflorum,* of *loveyly hemsleya,* of *Sambucus nigra,* of *Phaseolus lunatus,* of *Centaurium,* of *Macrocystis pyrifera,* of *Turnera diffusa,* of *Anemarrhena asphodeloides,* of *Portulaca pilosa,* of *Humulus lupulus,* of *Coffea arabica,* of *Ilex paraguariensis,* or of *Globularia cordifolia,* of *Albizzia julibrissin,* of *Oxydendron arboretum,* of *Zingimber zerumbet smith,* of *Astragalus membranaceus,* of *Atractylodes macrocephalae, of Plantago lanceolata, of Leontopodium alpinum, of Mirabilis jalapa,* of *Marrubium vulgare,* or of orchids.

The compositions of the present invention may include one or more peptides, including, without limitation, di-, tri-, tetra-, penta-and hexapeptides and their derivatives. According to a particular embodiment, the concentration of the additional peptide, in the composition, ranges from 1×10⁻⁷% and 20%, preferably from 1×10⁻⁶% and 10%, preferably between 1×10⁻⁵% and 5% by weight.

According to the present invention, the term "peptide" refers to peptides containing 10 amino acids or less, their derivatives, isomers and complexes with other species such as a metal ion (e.g. copper, zinc, manganese, magnesium, and others). The term "peptides" refers to both natural peptides and synthetic peptides. It also refers to compositions that contain peptides and which are found in nature, and/or are commercially available.

Suitable dipeptides for use herein include but are not limited to Carnosine (βAH), YR, VW, NF, DF, KT, KC, CK, KP, KK, TT, PA, PM or PP.

Suitable tripeptides for use herein include, but are not limited to RKR, HGG, GKH, GGH, GHG, KFK, KAvaK, KβAK, KAbuK, KAcaK, KPK, KMOK, KMOzK (MOz being a di-oxygenated sulfoxide methionine), KVK, PPL, PPR, SPR, QPA, LPA or SPA.

Suitable tetrapeptides for use herein include but are not limited to RSRK (SEQ ID NO: 1), GQPR (SEQ ID NO: 2), KTFK (SEQ ID NO: 3), KTAK (SEQ ID NO: 4), KAYK (SEQ ID NO: 5) or KFYK (SEQ ID NO: 6).

Suitable pentapeptides include, but are not limited to KTTKS (SEQ ID NO: 7). Suitable hexapeptides include but are not limited to GKTTKS (SEQ ID NO: 8) and VGVAPG (SEQ ID NO: 9).

Other suitable peptides for use herein include, but are not limited to: lipophilic derivatives of peptides, preferably palmitoyl (Pal) derivatives, and metal complexes as aforementioned (e.g. copper complex of the tripeptide HGG). Preferred dipeptide include for example N-Palmitoyl-β-Ala-His, N-Acetyl-Tyr-Arg-hexadecylester (Calmosensine^{™}, Idealift^{™} from Sederma), Pal-RT or Pal-KT (Sederma). Preferred tripeptide derivatives include for example Pal-GKH and Pal-GHK (from Sederma), the copper derivative of HGG (Lamin^{™} from Sigma), Lipospondin (N-Elaidoyl-KFK) and its analogs of conservative substitution, N-Acetyl-RKR-NH₂ (Peptide CK+), N-Biot-GHK (from Sederma), Pal-KAvaK, Pal-KβAlaK, Pal-KAbuK, Pal-KAcaK, or Pal-KMO₂K (Matrixyl^{®}synthe'6^{®} from Sederma), PalKVK (Syn-Coll^{™} of DSM), and derivatives thereof.

Here can also be cited the anti-aging tripeptides of general formula X-Pro^{∗}-Pro^{∗}-Xaa-Y disclosed in WO2015181688 with Xaa selected from Leu, Arg, Lys, Ala, Ser, and Asp; at the N terminal end, X selected from H, -CO-R₁ and -SO₂-R₁ and at the C terminal end Y selected from OH, OR₁, NH₂, NHR₁ or NR₁R₂; R₁ and R₂ being, independently from each other, selected from an alkyl, aryl, aralkyl, alkylaryl, alkoxy et aryloxy group, that can be linear, branched, cyclic polycyclic, saturated, unsaturated, hydroxylated, carbonylated, phosphorylated and/or sulfured, said group having or not an O, S and/or N heteroatom in its skeleton and Pro* corresponding to a Proline, analog or derivative thereof; comprising for example Myr-PPL-OH and Myr-PPR-OH.

Here can further be cited also the propigmenting and/or pro-mec dipeptides and tripeptides of general formula X-(Xaa₁)n-Pro^{∗}-Xaa₂-Y disclosed in WO2014/080376, with n=0, 1 or 2, Xaa₁ an hydrophobic aminoacid selected from Ala, Val, Met, Leu, Iso, Phe, Pro, and analogs and derivatives thereof; or a polar aminoacid selected from Ser, Thr, Tyr, Asp, Glu and analogs and derivatives thereof; and when n=2 the two aminoacids Xaa₁ being the same or different; Xaa₂ being an hydrophobic aminoacid selected from Ala, Val, Met, Leu, Iso, Phe, and analogs and derivatives thereof, or a basic aminoacid selected from Arg, Lys, His, and analogs and derivatives thereof; at the N terminal end X being selected from H, -CO-R₁ and -SO₂-R₁; at the C terminal end Y being selected from OH, OR₁, NH₂, NHR₁ or NR₁R₂; R₁ and R₂ being, independently from each other, selected from an alkyl, aryl, aralkyl, alkylaryl, alkoxy et aryloxy group, that can be linear, branched, cyclic polycyclic, saturated, unsaturated, hydroxylated, carbonylated, phosphorylated and/or sulfured, said group having or not an O, S and/or N heteroatom in its skeleton and Pro* corresponding to a Proline, analog or derivative thereof; comprising for example the following peptides Pal-SPR-OH, Pal-PPR-OH, Pal-QPA-OH, Pal-LPAOH, Myr-SPA-OH, Pal-PM-OH, Pal-PA-OH and Pal-PP-OH.

Suitable tetrapeptide derivatives for use according to the present invention include, but are not limited to, Pal-GQPR (SEQ ID NO: 10) (from Sederma) and Pal-KTFK (SEQ ID NO: 11) or Ela-KTFK (SEQ ID NO: 12), Ela-KTAK (SEQ ID NO: 13), Ela-KAYK (SEQ ID NO: 14) or Ela-KFYK (SEQ ID NO: 15). Suitable pentapeptide derivatives for use herein include, but are not limited to, Pal-KTTKS (SEQ ID NO: 16) (available as Matrixyl^{®} from Sederma), Pal-YGGFXaa (SEQ ID NO: 17) with Xaa being Leu or Pro, or mixtures thereof. Suitable hexapeptide derivatives for use herein include, but are not limited to, Pal-VGVAPG (SEQ ID NO: 18), Pal-GKTTKS (SEQ ID NO: 19), Pal-HLDIIXaa with Xaa being Trp, Phe, Tyr, Tic, 7-hydroxy-Tic ou Tpi (SEQ ID NO: 20) and derivatives thereof. The mixture of Pal-GHK and Pal-GQPR (SEQ ID NO: 10) (Matrixyl^{®} 3000, Sederma) can also be mentioned.

The preferred compositions commercially available containing a tripeptide or a derivative include Biopeptide-CL^{™}, Maxilip^{™}, Biobustyl^{™}, Procapil^{™} and Matrixyl^{®}synthe'6^{®} of Sederma. The compositions commercially available preferred sources of tetrapeptides include Rigin^{™}, Eyeliss^{™}, Matrixyl^{®} Reloaded and Matrixyl 3000^{®} which contain between 50 and 500 ppm of Pal-GQPR (SEQ ID NO: 10) and an excipient, proposed by Sederma.

The following marketed peptides can be mentioned as well as additional active ingredients:
- Vialox^{™} (INCI name = Pentapeptide-3 (synthetic peptide comprising alanine, arginine, isoleucine, glycine and proline)), Syn-ake^{™} (β-Ala-Pro-Dab-NH-Bzl) or Syn-Coll^{™} (Pal-Lys-Val-Lys-OH) marketed by Pentapharm;
- Argireline^{™} (Ac-Glu-Glu-Met-Gln-Arg-Arg-NH₂ (INCI name = Acetyl hexapeptide-3) (SEQ ID NO: 21), Leuphasyl^{™} (Tyr-D-Ala-Gly-Phe-Leu) (SEQ ID NO: 22), Aldenine^{™} (Gly-His-Lys), Trylagen^{™} (INCI name = Pseudoalteromonas Ferment Extract, Hydro lyzed Wheat Protein, Hydro lyzed Soy Protein, Tripeptide-10 Citrulline (reaction product of Citrulline and Tripeptide-10 (synthetic peptide constituted of aspartic acid, isoleucine and lysine)), Tripeptide-1), Eyeseryl^{™} (Ac-(3-Ala-His-Ser-His)(SEQ ID NO: 23), Serilesine^{™} (Ser-Ile-Lys-Val-Ala-Val) (SEQ ID NO 24) or Decorinyl^{™} (INCI name: Tripeptide-10 Citrulline = reaction product of Citrulline and Tripeptide-10 (synthetic peptide constituted of aspartic acid, isoleucine and lysine) marketed by Lipotec;
- Collaxyl^{™} (Gly-Pro-Gln-Gly-Pro-Gln (SEQ ID NO 25)) or Quintescine^{™} (Cys-Gly) marketed by Vincience;
- Cytokinol^{™}LS (casein hydrolysate) marketed by Les Laboratoires Serobiologiques/Cognis;
- Kollaren^{™} (Gly-His-Lys), IP2000^{™} (Pal-Val-Tyr-Val) or Meliprene^{™} (INCI name = Monofluoroheptapeptide-1: reaction product of acetic acide and a synthetic peptide comprising arginine, glycine, glutamic acid, histidine, norleucine, p-fluorophenylalanine and tryptophan) marketed by l'lnstitut Européen de Biologie Cellulaire;
- Neutrazen^{™} (Pal-His-D-Phe-Arg-NH₂) marketed by Innovations; or
- BONT-L-Peptide^{™} (INCI name = Palmitoyl Hexapeptide-19: reaction product of palmitic acid and Hexapeptide-19 (synthetic peptide constituted of asparagine, aspartic acid, lysine and methionine), Timp-Peptide^{™} (INCI name = Acetyl Hexapeptide-20: reaction product obtained by acetylation of Hexapeptide-20 (synthetic peptide constituted of alanine, glycine, lysine, valine and proline) or ECM Moduline^{™} (INCI name = Palmitoyl Tripeptide-28: reaction product of palmitic acid and Tripeptide-28 (synthetic peptide constituted of arginine, lysine and phenylalanine) marketed by Infinitec Activos.

More specifically, according to the invention the alkyl-phthalide plant extract may be combined with at least one of compounds selected from compounds of the vitamin B3, compounds such as niacinamide or tocopherol, retinoid compounds such as retinol, hexamidine, α-lipoic acid, resveratrol or DHEA, hyaluronic acid, peptides, in particular N-acetyl-Tyr-Arg-O-hexadecyl ester, Pal-VGVAPG (SEQ ID NO: 18), Pal-KTTKS (SEQ ID NO: 16), Pal-GHK, Pal-KMOzK and Pal-GQPR (SEQ ID NO: 10), which are widely used active ingredients in topical cosmetic or dermopharmaceutical compositions.

More specifically, and advantageously, it is possible to associate with the treatment according to the invention another slimming active agent in complement and reinforcement of the slimming active ingredient according to the invention based on alkyl phthalides.

This additional slimming agent may be a compound especially of the family of methylxanthines, such as, for example, caffeine, theophylline, theobromine or aminophylline. These compounds are known for their lipolytic actions and also for their draining action (promoting the elimination of water) and stimulating action (increasing the metabolic activity of the cells). They may be brought in the form of a plant material.

Mention may also be made, by way of examples, of phosphodiesterase inhibitor compounds, alpha-2 blocking compounds capable of blocking alpha-2 receptors on the surface of adipocytes, beta-adrenergic agonists and antagonists (eg alverine or a organic or inorganic salt compound of alverine such as alverine citrate), compounds inhibiting LDL or VLDL receptor synthesis, inhibitors of fatty acid synthesis enzymes, such as acetyl CoA carboxylase or fatty acid synthetase or cerulenin, beta-receptor- and/or G-proteins stimulating compounds, glucose transport blockers, such as serine or rutin, neuropeptide Y (NPY) antagonists capable of blocking NPY receptors on the surface of adipocytes, AMPc and its cosmetically acceptable derivatives, adenylate cyclase activating agents such as forskolin, fatty acid transport modifying agents, lipolytic peptides and lipolytic proteins, such as peptides or proteins such as peptides derived from the parathyroid hormone.

Other examples of slimming agents known for their lipolytic property that can be used in the context of the present invention include, without limitation, extracts of plant or marine origin:
- Among the plant extracts, mention may be made especially of climbing Ivy extract *(Hedera Helix), Bupleurum chinensis,* arnica (*Arnica Montana L),* rosemary *(Rosmarinus officinalis N),* marigold *(Calendula officinalis),* sage *(Salvia officinalis L),* ginseng *(Panax ginseng),* ginko biloba, St. John's wort *(Hyperycum Perforatum*), fragon *(Ruscus aculeatus L), Filipendula ulmaria* L, orthosiphon *(Orthosiphon Stamincus Benth),* algae *(Fucus Vesiculosus),* birch *(Betula alba),* cola nut *(Cola Nipida),* green tea, chestnut, bamboo, *Centella asiatica,* heather, fucus, willow, mouse-ear hawkweed, extracts of escin, extracts of cangzhu, extracts of *Chrysanthellum indicum,* extracts of plants of the genus *Armeniacea, Atractylodis Platicodon, Sinommenum, Pharbitidis, Flemingia,* coleus extracts such as *C. Forskohlii, C blumei, C. esquirolii, C. scutellaroides, C. xanthantus* and C. *Barbatus,* such as the extract of roots of *Coleus Barbatus,* extracts of Ballote, extracts of *Guioa, Davallia, Terminalia, Barringtonia, Trema, Antirobia, Cecropia,* of argan tree, dioscorees rich in diosgenin, including *Dioscorea opposita* or *mexican* or *villosa .*
- As extracts of marine origin, mention may be made of extracts of algae or of phytoplankton such as an extract of *Laminaria digitata* or rhodysterol.

Zerumbone, for example extracted from *Zingiber zerumbet smith,* and marketed, for example, in the active ingredients of Sederma: Legance^{™}, Zingerslim^{™} and Intenslim^{™} can also advantageously be combined with the phthalide(s) according to the invention. The following combinations of actives, marketed by Sederma, for slimming and silhouette sculpting can be also cited: Vexel^{™}, Coaxel^{™}, Cyclolipase^{™}, Lipocare^{™}, Redulite^{™}, Bodyfit^{™}, Unislim^{™}, Esculoside^{™}, Phytotonin^{™}, and Lipoline^{™}.

Other complementary active agents may also be used according to the invention chosen from: the active agents acting on the microcirculation (vasculoprotectors or vasodilators), such as flavonoids, ruscogenins, natural or synthetic esculosides, escin, nicotinates, heperidin methyl chalcone, butcher's broom, essential oils of lavender or rosemary, extracts of *Ammi visnaga;* firming and/or antiglycant active agents such as extracts of *Centella asiatica* and *Siegesbeckia,* silicon, amadorin, ergothioneine and derivatives thereof, hydroxystilbenes and derivatives thereof, in particular resveratrol, plant extracts of the family of the Ericaceae, in particular blueberry extracts *(Vaccinium angustifollium*), vitamin C and its derivatives and retinol and its derivatives.

The present invention also provides a slimming cosmetic topical treatment method comprising topically applying to the face and/or body of a subject in need thereof an effective amount of an extract *of Apium graveolens* seeds obtained by COz supercritical extraction comprising at least 50% by weight of a mixture of alkyl phthalides consisting of sedanenolide, sedanolide and 3-n-butylphthalide- in a physiologically acceptable excipient. The method of treatment according to the invention more specifically allows a lipolytic and anti-lipogenic action, making it possible to prevent and reduce the formation of fatty clusters.

"Topical treatment" or "topical use" means according to the invention, an application that is intended to act where it is applied.

A composition according to the invention may be applied locally to targeted areas, for example using a cannula type of applicator or wipes.

The "effective" amount depends on various factors, such as the age, the severity of the disorder and the administration mode, etc. An effective amount means a non-toxic amount enough to achieve the desired effect.

All percentages and ratios used herein are by weight of the total composition and all measurements are made at 25°C unless it is otherwise specified.

By way of example, for cosmetic treatment of the body, the European Cosmetics Directive has fixed a standard amount of application of a lotion of 0.5 mg cm²/day/person and for cosmetic treatment of the face a cream at 2.72 mg/cm²/day/person.

According to other specific features, the cosmetic treatment method according to the invention can be combined with one or more other treatment methods targeting the skin such as lumino-therapy, heat or aromatherapy treatments.

According to the invention, devices with several compartments or kits may be proposed to apply the method described above which may include for example and non-restrictively, a first compartment containing a composition comprising the alkyl-phthalide plant extract according to the invention, and in a second compartment another active ingredient and/or excipient, the compositions contained in the said first and second compartments in this case being considered to be a combination composition for simultaneous, separate or stepwise use in time, particularly in one of the treatment methods recited above.

### A) Example of preparation of an Apium graveolens seeds extract that can be used in the context of the invention

The *Apium graveolens* seeds are ground to a particle size of powder around 800µm. This powder is then extracted with supercritical COz under 90 bars (90.10⁵ Pa) pressure and at 40°C. The residual water that may be present in the final extract is then removed if necessary (for example by decantation, vacuum evaporation, and lyophilization). The extraction yield is around 2.5%. The extract is in the form of an oily liquid, clear to slightly opalescent, colorless to pale yellow.

The resulting extract is passed on Ultra High Performance Liquid Chromatography (UHPLC) on a C18 column with as mobile phase a water/acetonitrile gradient, to dose the various phthalides.

This analysis confirms the presence of 71% of total phthalides in the extract comprising the 3-alkyl phthalides: 10% of 3-n-butylphthalide, 30% of sedanolide and 60% of sedanenolide by weight relative to the total phthalides. The extract can be used pure or diluted.

### B) Formulation of an active ingredient that can be used according to the invention

The active ingredient is a composition comprising the extract of *Apium graveolens* seeds obtained according to A) above dissolved in a matrix forming a physiologically acceptable medium. This active ingredient is particularly intended for the cosmetics industry for the preparation of cosmetics, creams, gels (See galenical examples at point D) below).

An extract obtained according to A) above can be diluted in any physiologically acceptable fatty excipient to reach at the end a concentration of 500ppm of alkyl-phthalides. Esterified oil is preferably used of Caprylic/Capric Triglyceride type for this dilution.

For example, and for the description of the galenic of point D) below, it is this dilution that has been preferably used. This constitutes the active ingredient that will be used itself preferably at between 1 and 5% in a cosmetic composition that can be applied on skin.

### C) In vitro evaluations

The slimming ability according to the invention was studied *in vitro* with the two tests presented below, carried out using a crude extract *of Apium graveolens* seed oil manufactured according to the method of production described in point A) above.

### 1) Evaluation of the lipolytic effect by measurement of glycerol releasing on human adipocytes.

Glycerol is a product of triglyceride hydrolysis. The stimulation of its production in the presence of an active agent therefore reflects an increase in lipolysis.

### Principle:

Human pre-adipocytes are seeded and induced to differentiate with a specific cocktail of inducers. To obtain mature adipocytes well loaded with triglycerides (TG), the cells are brought into contact with the extract according to the invention in a maintenance culture medium containing 2% desteroided FBS (test).

The supernatants are then recovered and the amount of glycerol released from the hydrolysis of the intracellular TG is measured at 48 hours using a commercial kit from Sigma. In parallel, a survival assay is carried out by Hoechst staining to quantify the number of cells.

Visual inspection is carried out before each recovery to verify the absence of toxicity.

### Results:

**Table 1: Measurement of the glycerol released on adipocyte after 48 hours of contact with the extract according to the invention**

| **Concentration of released glycerol** | | **Variation** |
|---|---|---|
| Control | | Reference |
| CO₂ extract of *Apium graveolens* seeds | 24 ppm | +**67%**; *p<0.01* |

These results show that the extract according to the invention increases the lipolysis of the adipocytes.

### 2) Evaluation of the anti-lipogenesis effect by measuring the inhibition of G3PDH on human adipocytes.

Glycerol-3-Phosphate Dehydrogenase (G3PDH) is an enzyme whose expression increases strongly during the differentiation of pre-adipocyte fibroblasts into adipocytes. Its inhibition will reduce therefore the amount of adipocytes and therefore the adipose tissue.

### Principle:

Human pre-adipocytes are induced to differentiate into mature adipocytes using a specific cocktail of inducers in the presence of the extract according to the invention. When the differentiating adipocytes are obtained, the cells are harvested and then ground, and the G3PDH is extracted from the supernatant. After centrifugation of the ground product, an activity assay is carried out using a spectrophotometer.

In parallel, a survival assay is carried out by Hoechst staining to quantify the number of cells.

Visual inspection is carried out before each recovery to verify the absence of toxicity.

### Results:

**Table 2: Measurement of G3PDH activity on adipocytes in the way of differentiating**

| **G3PDH activity** | | **Variation** |
|---|---|---|
| Control | | Reference |
| CO₂ extract of *Apium graveolens* seeds | 24 ppm | **-28%;** *p<0.01* |
| | 32 ppm | **-60%;** *p<0.01* |
| | 40 ppm | ***-79%;** p<0.01* |
| | 48 ppm | ***-92%;** p<0.01* |

These results show that the extract according to the invention slows down the differentiation of the adipocytes significantly with a dose-dependent effect of 24 to 48 ppm.

### D) Galenic

Various cosmetic formulations that can be used for implementing the present invention are described below. Additional active ingredients, coming when appropriate in support and/or in addition to the activity of the active ingredient according to the invention can be added in the correct formulation part according to their hydrophobic or hydrophilic nature. These ingredients can be of any category according to their(s) function(s), the desired end, and site of application (body, face, neck, chest, hands), and the targeted user, for example, an anti-aging, anti-wrinkles, sunscreen, antioxidant, moisturizing, firming, against rednesses, against stretch marks, tightening or lifting, soothing, smoothing.

**Active ingredient according to the invention** used in the galenic formulations given below: COz supercritical extract of *Apium graveolens* seeds included in an ester oil of Caprylic/Capric Triglyceride type so as to be at the end at 500 ppm of total alkyl phthalides.

This ingredient is preconized between 0.1 and 10%, preferably between 2 and 5%, according to the more or less pronounced effect expected.

### Example 1: Slimming cream gel

| **Ingredient** | % | **INCI name** |
|---|---|---|
| **Part A** | | |
| H₂O | Qsp 100 | Water |
| Optasense G83^{™} | 0.40 | Carbomer |

| **Part B** | | |
|---|---|---|
| Crodacol CS 90^{™} | 2.00 | Cetearyl Alcohol |
| Crodamol GTCC ^{™} | 3.00 | Caprylic/Capric Triglyceride |

| **Part C** | | |
|---|---|---|
| Butylene glycol | 4.00 | Butylene glycol |
| Phenoxyethanol | 1.00 | Phenoxyethanol |

| **Part D** | | |
|---|---|---|
| Optasense G82 ^{™} | 0.20 | Acrylic Acid/Alkylmethacrylate Copolymer |
| DC 200 5 cps^{™} | 3.00 | Dimethicone |

| **Part E** | | |
|---|---|---|
| Potassium sorbate | 0.10 | Potassium Sorbate |

| **Part F** | | |
|---|---|---|
| H₂O | 6.00 | Water |
| NaOH 30% | 0.60 | Sodium Hydroxide |

| **Part G** | | |
|---|---|---|
| Supercritical CO₂ extract *of Apium graveolens* seeds according to the invention | 3.00 | / |

| **Part H** | | |
|---|---|---|
| Tween 20^{™} | 1.00 | Polysorbate 20 |
| Perfume | 0.10 | Fragrance |

### Example 2: Slimming massage cream

| **Ingredient** | **%** | **INCI name** |
|---|---|---|
| **Part A** | | |
| H₂O | Qsp100 | Water |
| Ultrez 10^{™} | 0.25 | Carbomer |

| **Part B** | | |
|---|---|---|
| Hydrolite-5 ^{™} | 5.00 | Pentylene Glycol |
| Phenoxyethanol | 1.00 | Phenoxyethanol |
| Keltrol CG-SFT ^{™} | 0.25 | Xanthan Gum |

| **Part C** | | |
|---|---|---|
| Brij S2^{™} | 0.40 | Steareth-2 |
| Brij S10^{™} | 1.20 | Steareth-10 |
| Crodafos CES^{™} | 4.00 | Cetearyl Alcohol & Dicetyl Phosphate & Ceteth-10 Phosphate |
| Crodamol CSO^{™} | 2.50 | Cetearyl Ethylhexanoate |
| BRB CM 56^{™} | 2.00 | Cyclohexasiloxane & Cyclopentasiloxane |
| Crodamol OSU^{™} | 7.00 | Diethylhexyl Succinate |
| Crodacol CS 90^{™} | 1.50 | Cetearyl Alcohol |

| **Part D** | | |
|---|---|---|
| Potassium sorbate | 0.10 | Potassium Sorbate |

| **Part E** | | |
|---|---|---|
| H₂O | 4.00 | Water |
| NaOH 30 % | 0.40 | Sodium Hydroxide |

| **Part F** | | |
|---|---|---|
| Supercritical CO₂ extract *of Apium graveolens* seeds according to the invention | 5.00 | / |

### Examples of ingredients that can be added to formulations in the appropriate phase (marketed by Sederma):

- INTENSLIM^{™}: slimming active ingredient corresponding to a synergistic combination of extracts obtained by *Globularia cordifolia* plant cell culture, *Zingiber zerumbet Smith* titrated in zerumbone and vegetable caffeine obtained by supercritical COz extraction.
- LEGANCE^{™}: anti-aging active, corresponding to a *Zingiber zerumbet Smith* extract obtained by COz supercritical in a water-soluble excipient and titrated in zerumbone ingredient. It is a global anti-aging ingredient for legs. It improves their appearance and comfort by reducing water retention, improving microcirculation and refining adipose tissue.
- REVIDRATE^{™}: active (Myristyl Phosphomalate) that in particular improves the cohesion of the epidermis and its hydration. 2% of this ingredient may for example be added to Phase C of the formulation.
- RIGIN^{™}: Palmitoyl-Gly-Gln-Pro-Arg peptide improving the elasticity and firmness of the skin, increasing hydration and smoothing the skin. 3% by weight of this ingredient may for example be added to the formulation in phase G.
- IDEALIFT^{™}: active ingredient - Butylene Glycol (and) Water (and) Sorbitan Laurate (and) Hydroxyethylcellulose (and) Acetyl Dipeptide-1 Cetyl Ester) who fights flaccidity of the face and improves resistance to gravity. 4% of this ingredient can for example be added to the phase F.
- BODYFIT^{™}: slimming/firming active ingredient comprising glaucine. It reduces the appearance of cellulite and helps to improve drainage and water distribution in the tissues. 3% by weight may be added for example to phase F of the formulation.
- MATRIXYL^{™}, MATRIXYL 3000^{™} and/or MATRIXYL synthe'6^{™}: anti-aging, anti-wrinkle based peptide(s) (as mentioned above) that help repair skin damage caused by aging.
- SUBLISKIN^{™}: active ingredient that moisturizes and smooths the skin while allowing it to resist to external aggressions (based on acetylated glucuronic acid oligosaccharides).

## Claims

1. Use of an extract of *Apium graveolens* seeds obtained by COz supercritical extraction comprising at least 50% by weight of a mixture of alkyl phthalides consisting of sedanenolide, sedanolide and 3-n-butylphthalide, for a topical cosmetic slimming treatment.

2. Use as claimed in claim 1, **characterized in that** said extract is obtained by COz supercritical extracting of crushed seeds between 75.10⁵ and 300.10⁵ Pa of pressure and between 30 and 80°C.

3. Use according to claim 2, **characterized in that** said extract is obtained by COz supercritical extracting of crushed seeds at 90.10⁵ Pa (90 bars) pressure and at 40°C.

4. Use according to one of claims 1 to 3, **characterized in that** said mixture of alkyl phthalides comprises at least 50% by weight of sedanenolide.

5. Use according to claim 4, **characterized in that** said mixture of alkyl phthalides comprises at least 70% by weight of sedanenolide.

6. Use according to anyone of claims 1 to 5, **characterized in that** the extract contains at least 65% by weight of said mixture of alkyl phthalides.

7. Use according to one of claims 1 to 4 for a lipolytic and anti-lipogenic treatment.

8. Use according to one of claims 1 to 5, **characterized in that** said extract is diluted in a physiologically acceptable medium.

9. Use according to any one of the preceding claims, for a treatment preventing and reducing the installation of unsightly fat clusters, "orange peel" effect and/or cellulite.

## Patentansprüche

1. Verwendung eines durch überkritische CO₂-Extraktion erhaltenen Extrakts aus Samen von *Apium graveolens,* der mindestens 50 Gew.-% eines Gemisches von Alkylphthaliden, bestehend aus Sedanenolid, Sedanolid und 3-n-Butylphthalid, umfasst, für eine topische kosmetische Abnehmbehandlung.

2. Verwendung nach Anspruch 1, **dadurch gekennzeichnet, dass** der Extrakt durch überkritische CO₂-Extraktion von zerkleinerten Samen bei einem Druck zwischen 75.10⁵ und 300.10⁵ Pa und zwischen 30 und 80 °C erhalten wird.

3. Verwendung nach Anspruch 2, **dadurch gekennzeichnet, dass** der Extrakt durch überkritische CO₂-Extraktion von zerkleinerten Samen bei einem Druck von 90.10⁵ Pa (90 bar) und bei 40 °C erhalten wird.

4. Verwendung nach einem der Ansprüche 1 bis 3, **dadurch gekennzeichnet, dass** das Gemisch von Alkylphthaliden mindestens 50 Gew.-% Sedanenolid umfasst.

5. Verwendung nach Anspruch 4, **dadurch gekennzeichnet, dass** das Gemisch von Alkylphthaliden mindestens 70 Gew.-% Sedanenolid umfasst.

6. Verwendung nach einem der Ansprüche 1 bis 5, **dadurch gekennzeichnet, dass** der Extrakt mindestens 65 Gew.% des Gemisches von Alkylphthaliden enthält.

7. Verwendung nach einem der Ansprüche 1 bis 4 für eine lipolytische und anti-lipogene Behandlung.

8. Verwendung nach einem der Ansprüche 1 bis 5, **dadurch gekennzeichnet, dass** der Extrakt in einem physiologisch unbedenklichen Medium verdünnt ist.

9. Verwendung nach einem der vorangehenden Ansprüche für eine Behandlung, die der Bildung von unansehnlichen Fettansammlungen, dem "Orangenhaut"-Effekt und/oder der Cellulite vorbeugt und diese verringert.

## Revendications

1. Utilisation d'un extrait de graines d'*Apium graveolens* obtenu par extraction au CO₂ supercritique comprenant au moins 50% en poids d'un mélange d'alkyl-phtalides constitué de sédanénolide, de sédanolide et de 3-n-butylphtalide, pour un traitement cosmétique topique amincissant.

2. Utilisation selon la revendication 1, **caractérisée en ce que** ledit extrait est obtenu par extraction au CO₂ supercritique de graines broyées entre 75.10⁵ (75 bars) et 300.10⁵ Pa (300 bars) de pression et entre 30 et 80°C.

3. Utilisation selon la revendication 2, **caractérisée en ce que** ledit extrait est obtenu par extraction au CO₂ supercritique de graines broyées à une pression de 90.10⁵ Pa (90 bars) et à 40°C.

4. Utilisation selon l'une des revendications 1 à 3, **caractérisée en ce que** ledit mélange d'alkyl-phtalides comprend au moins 50% en poids de sédanénolide.

5. Utilisation selon la revendication 4, **caractérisée en ce que** ledit mélange d'alkylphtalides comprend au moins 70% en poids de sédanénolide.

6. Utilisation selon l'une quelconque des revendications 1 à 5, **caractérisée en ce que** l'extrait contient au moins 65 % en poids dudit mélange d'alkyl-phtalides.

7. Utilisation selon l'une des revendications 1 à 4, pour un traitement lipolytique et antilipogenèse.

8. Utilisation selon l'une des revendications 1 à 5, **caractérisée en ce que** ledit extrait est dilué dans un milieu physiologiquement acceptable.

9. Utilisation selon l'une quelconque des revendications précédentes, pour un traitement permettant de prévenir et diminuer l'installation d'amas graisseux disgracieux, de « peau d'orange », et/ou de cellulite.
